# EUROPEAN PATENT APPLICATION

(11) **EP 4 480 514 A1**
(43) Date of publication of application: **25.12.2024**
(21) Application number: 23181055.7
(22) Date of filing: 22.06.2023
(51) Int. Cl.: A61M 1/06, A61B 5/00

(54) **MILK EXPRESSION DEVICE PUMPING OPERATION CONTROL**

(71) Applicant: Koninklijke Philips N.V., 5656 AG Eindhoven (NL)
(72) Inventor: PAULUSSEN, Elvira Johanna Maria, Eindhoven (NL); SEGAAR, Lucja Elzbieta, Eindhoven (NL); VAN DEN DUNGEN, Wilhelmus Andreas Marinus Arnoldus Maria, Eindhoven (NL); KOOIJMAN, Gerben, Eindhoven (NL); BOURQUIN, Yannyk Parulian Julian, Eindhoven (NL); FERNANDO, Shakith Devinda, Eindhoven (NL)
(74) Representative: Philips Intellectual Property & Standards

(57) **Abstract**

Provided is a controller (3A) for controlling pumping operation of a milk expression device (3). The controller comprises an input (34) for receiving an input signal indicative of a user's milk ejection reflex. The controller is configured to generate, in response to the input signal being received, an output for controlling pumping operation of the milk expression device to implement an expression pressure profile comprising a non-zero baseline under-pressure. Further provided is a milk expression system comprising the controller, and a method and related computer program for controlling pumping operation of a milk expression device.

## Description

### FIELD OF THE INVENTION

The invention relates to a controller for controlling pumping operation of a milk expression device, e.g. a breast pump. The invention also relates to a milk expression assembly comprising the milk expression device and the controller. The invention further provides a method of operating such a milk expression device, and a related computer program.

### BACKGROUND OF THE INVENTION

Human breast milk is the most important nutrient for newborns and infants. Due to difficult breastfeeding experiences, many mothers opt to stop breastfeeding early. Only 40% of all mothers exclusively breastfeed in the first 6 months after birth. The most prevalent reason for stopping exclusive breastfeeding is the perception of insufficient milk supply, referring to Gatti, L., Journal of nursing scholarship, 2008, 40, 355-363. Another problem mothers tend to face is lactiferous duct blockages, in other words milk duct blockages. Such blockages can cause mastitis.

To provide the best nutrition to their babies, mothers may express milk using a breast pump. The expressed milk can be stored and given to the baby at a later stage. Typically, the breast is placed into a funnel, also known as a breast shield, and a vacuum is applied such that milk is extracted. Breast pumps tend to have a stimulation mode to activate the mother's milk ejection reflex, and an expression mode.

Transition from the stimulation mode to the expression mode may be time-based. In other words, the transition may be automatically implemented a fixed time after switching on. Alternatively, the transition may be manually controlled by pushing a button.

### SUMMARY OF THE INVENTION

Whilst attempts have been made to use detection of the milk ejection reflex to enable automatic switching between different modes, challenges have been encountered in terms of providing appropriate pressure-related parameter(s), e.g. pumping parameter(s), in response to detection of the milk ejection reflex. Moreover, it would be desirable to provide more reliable detection of the milk ejection reflex, and/or to provide measures that mitigate consequences of misidentification of the milk ejection reflex.

The invention is defined by the claims.

According to examples in accordance with an aspect of the invention, there is provided a controller for controlling pumping operation of a milk expression device, wherein the controller comprises an input for receiving an input signal indicative of a user's milk ejection reflex, and wherein the controller is configured to generate, in response to the input signal being received, an output for controlling pumping operation of the milk expression device to implement an expression pressure profile comprising a non-zero baseline under-pressure.

The present invention is partly based on the insight that complete release of a nipple tissue area, due to an expression pressure profile employing a zero baseline under-pressure, upon a milk ejection reflex taking place provides sub-optimal mimicry of an infant's sucking behavior. This is because the infant tends not to completely release an under-pressure generated by their sucking when milk is being released.

For this reason, a non-zero baseline under-pressure is employed in the expression pressure profile implemented via the controller's control over the pumping operation of the milk expression device, e.g. breast pump, in response to an input signal indicative of the user's milk ejection reflex.

By providing enhanced mimicry of an infant's sucking behavior in response to milk ejection reflex in this way, milk extraction from the mother's breast using the milk expression device may be enhanced.

In some embodiments, the controller is configured to generate an initial output for controlling the milk expression device to implement an initial pressure profile, such as a stimulation pressure profile.

It is noted that the stimulation pressure profile may stimulate the user of the milk expression device to experience milk ejection reflex.

The initial output may, for example, be generated at the beginning of a session of use of the milk expression device, in other words breast pump session.

In some embodiments, when the input signal indicative of the milk ejection reflex is no longer received, for example due to milk flow falling below a predefined threshold, the controller may be configured to control the milk expression device to implement, e.g. revert to implementing, the initial pressure profile.

It is noted that the term "baseline under-pressure" may refer to a minimum under-pressure, e.g. minimum vacuum, employed during the expression pressure profile.

The baseline under-pressure may be the pressure in the expression pressure profile that comes the closest to atmospheric pressure, but does not reach atmospheric pressure due to being a non-zero baseline under-pressure.

In at least some embodiments, the expression pressure profile comprises cycling of pressure between the non-zero baseline under-pressure and a maximum/upper level of under-pressure, e.g. a maximum vacuum.

In some embodiments, the controller is configured to generate the output in response to a milk delivery signal relevant to milk delivery from the user's breast.

In such embodiments, the milk delivery signal may be relevant to a milk flow and/or a milk amount from the user's breast.

The milk delivery signal may provide a relatively straightforwardly implementable way of detecting the milk ejection reflex. To this end, a sensor system that generates the milk delivery signal may include a milk delivery sensor, for example an optical milk delivery sensor and/or an acoustic milk delivery sensor, for sensing milk delivery.

The optical milk delivery sensor may operate using any suitable detection principle. For example, the optical milk delivery sensor may employ laser doppler, laser speckle, doppler optical coherence tomography (OCT), photoplethysmography (PPG), visible/near-infrared laser, LED and/or super luminescent diode (SLD) technology.

In some embodiments, the sensing is based on detecting interruption of a light beam. Alternatively, it may be based on measurement of a change in reflection or scattering of light back from a layer of milk.

The acoustic milk delivery sensor may, for example, include a microphone for detecting a sound indicative of milk transport and/or collection.

In some embodiments, the controller is configured to generate a milk delivery-based control signal for setting a level of the non-zero baseline under-pressure based on the milk delivery signal. Thus, the non-zero baseline under-pressure can be aligned with milk delivery, for instance such that a deeper non-zero baseline under-pressure is employed when milk delivery is higher and/or increasing, and a diminished non-zero baseline under-pressure is employed when milk delivery is lower and/or decreasing.

In some embodiments, the controller is configured to generate the output in response to the milk delivery signal being indicative of a milk delivery parameter reaching or passing a predetermined threshold for at least a predetermined time period. In this way, the risk of false identification of the milk ejection reflex based on milk delivery may be lessened.

In some embodiments, the milk delivery parameter comprises a milk flow rate and/or a change in milk flow rate.

The controller may be configured to generate, in response to the input signal being received, an output signal for causing the non-zero baseline under-pressure to deepen compared to an initial baseline under-pressure provided prior to the input signal being received. In this way, milk flow may be promoted following onset of the milk ejection reflex.

The initial baseline under-pressure may be included in the initial pressure profile, e.g. stimulation pressure profile.

In some embodiments, the controller is configured to implement the stimulation pressure profile at the beginning of a session of use of the milk expression device and/or is configured to implement the stimulation pressure profile at one or more intermediate stages during the session, e.g. between a first input signal indicative of milk ejection reflex being no longer received and a second input signal indicative of a subsequent milk ejection reflex being received.

The controller may be configured to generate, in response to the input signal being received, output signal data for causing the non-zero baseline under-pressure to be maintained during a main period during which the expression pressure profile is implemented, and then to diminish during an end period that follows the main period.

By the non-zero baseline under-pressure diminishing during the end period relative to in the main period, the change in the baseline under-pressure may reflect decreasing milk flow that occurs as the milk ejection reflex is ending.

In some embodiments, the controller is configured to generate the output in response to the input signal being indicative of the milk ejection reflex being maintained for at least a predetermined duration. This may assist to minimize the risk of false identification of the milk ejection reflex.

The controller may be configured to generate, during the predetermined duration, a control output for controlling pumping operation of the milk expression device to implement an adaptation pressure profile in which one or more pressure-related parameters are progressively adjusted towards the expression pressure profile comprising the non-zero baseline under-pressure.

Thus, the milk expression device may continue to pump during suspected milk ejection reflex onset and adopt parameter(s) towards the expression pressure profile, whose parameters, and in particular the non-zero baseline under-pressure, may be adapted for established milk ejection reflex.

This may be superior to updating the pressure-related/pumping parameters upon the input signal being initially received but immediately resuming the initial pressure profile, e.g. stimulation pressure profile, should the input signal not be maintained for the predetermined duration. This is because the progressive adjustment towards the expression pressure profile may assist to minimize the risk that changes to the pressure-related/pumping parameters, e.g. and associated disturbance of pump rhythm, negatively influence stimulus received by the breast.

As an alternative or in addition to the controller generating the output in response to the milk delivery signal, the controller may be configured to generate the output in response to a milk duct dimension signal relevant to a milk duct dimension.

In such embodiments, milk duct dilation that occurs with onset of the milk ejection reflex may be utilized in controlling the pumping operation of the milk expression device to implement the expression pressure profile.

To this end, a sensor system that generates the milk duct dimension signal may include a milk duct sensor, for example an optical milk duct sensor, for sensing milk duct dimensions.

The optical milk duct sensor may operate using any suitable detection principle. In some embodiments, the milk duct sensor comprises a single-photon avalanche diode (SPAD) sensor.

In some embodiments, the input signal comprises a skin characteristic signal.

To this end, a sensor system that generates the skin characteristic signal may include a skin sensor for sensing one or more skin characteristics relevant to milk ejection reflex, such as a skin perfusion, skin temperature, skin impedance, and/or skin deformation.

Such a skin sensor may include an optical skin sensor and/or a non-optical skin sensor.

In some embodiments, the controller is configured to generate the output in response to the milk duct dimension signal being indicative of a milk duct dimension parameter reaching or passing a given threshold.

Alternatively or additionally, the controller may be configured to generate a duct dimension-based control signal to set a magnitude of the non-zero baseline under-pressure based on the milk duct dimension signal.

In some embodiments, the milk duct dimension parameter comprises a milk duct dimension and/or a change, e.g. increase, in a milk duct dimension.

The controller may be configured to generate, in response to the input signal, a further output for controlling one or more mechanical vibration units for vibrating at least part of the user's breast. This may assist to alleviate blocked duct(s), for example with the vibration of the vibration unit(s) operating in tandem with the non-zero baseline under-pressure to assist with unblocking duct(s).

In some embodiments, the further output for controlling the mechanical vibration unit(s) is based on the milk duct dimension signal and/or on one or more parameters of the expression pressure profile.

The vibration unit(s) may be activated by the controller for regions of the breast determined to include blocked ducts.

The controller may be configured to adjust the expression pressure profile according to a duct blockage level, for example fully blocked or partially blocked.

Alternatively or additionally, the controller may be configured to control the mechanical vibration unit(s) to vibrate, e.g. massage, the breast when the expression pressure profile is being implemented.

In this manner, milk in the blocked ducts can be suctioned and drained out.

In some embodiments, the controller is configured to adapt vibrating provided by the vibration unit(s) based on the milk duct dimension signal, for example as a diameter of a blocked duct changes.

Such adaptation of the vibrating, e.g. vibrating level and/or vibrating frequency, provided by the vibration unit(s) can be implemented in real time, for example together with adjustment of parameter(s) of the expression pressure profile.

In some embodiments, the controller comprises a further input for receiving a further input signal indicative of a maximum milk flow and/or a degree of discomfort experienced by the user. In such embodiments, the controller may be configured to generate a control signal to set an upper level of the under-pressure in the expression pressure profile based on the further input signal.

This may assist to make milk expression using the milk expression device more comfortable for the user.

According to another aspect there is provided a milk expression system comprising a milk expression device and the controller according to any of the embodiments described herein for controlling pumping operation of the milk expression device.

In some embodiments, the milk expression system comprises a sensor system for generating the input signal indicative of the milk ejection reflex.

The sensor system may comprise one or more of a milk delivery sensor for sensing milk delivery, a milk duct sensor for sensing a milk duct dimension, and a skin sensor for sensing one or more skin characteristics relevant to milk ejection reflex.

According to a yet another aspect there is provided a method for controlling pumping operation of a milk expression device, the method comprising: receiving an input signal indicative of a milk ejection reflex; and generating, in response to the input signal being received, an output for controlling pumping operation of the milk expression device to implement an expression pressure profile comprising a non-zero baseline under-pressure.

According to a further aspect there is provided a computer program comprising computer program code configured to cause a controller, when said code is executed on said controller, to execute the method according to any of the embodiments described herein.

One or more non-transitory computer readable media may be provided, which non-transitory computer readable media have a computer program stored thereon, with the computer program comprising computer program code which is configured, when the computer program is run on a controller, to cause the controller to implement the method according to any of the embodiments described herein.

The controller can be included in a milk expression device, e.g. breast pump, and/or can be in a user device, for example a smart phone or tablet computer, separate from such a milk expression device, and/or in a cloud-based server.

Embodiments described herein in relation to the controller may be applicable to the milk expression system, method and computer program, embodiments described herein in relation to the milk expression system may be applicable to the controller, method and computer program, embodiments described herein in relation to the method may be applicable to the controller, milk expression system and computer program, and embodiments described herein in relation to the computer program may be applicable to the controller, milk expression system and method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 schematically depicts a milk expression system according to an example;
FIG. 2 provides a detailed view of part of a milk expression system according to an example;
FIG. 3 provides a flowchart of a method according to a first example;
FIG. 4 provides a graph of milk flow rate vs time when a milk ejection reflex takes place;
FIG. 5 provides a graph of milk flow rate vs time in the case of a false onset milk ejection reflex;
FIGs. 6 to 8 show example pressure profiles provided by a milk expression device;
FIG. 9 provides a flowchart of a method according to a second example;
FIG. 10 provides a graph of duct diameter vs time for a mother;
FIG. 11 provides a graph of duct diameter vs time for a mother different from the mother whose duct diameter vs time is plotted in FIG. 10;
FIG. 12 schematically depicts a sensor system according to an example;
FIG. 13 schematically depicts a sensor system according to another example;
FIG. 14 provides a flowchart of a method according to a third example;
FIG. 15 provides a flowchart of a method according to a fourth example;
FIG. 16 shows a breast-receiving funnel according to an example; and
FIG. 17 provides a flowchart of a method according to a fifth example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a controller for controlling pumping operation of a milk expression device. The controller comprises an input for receiving an input signal indicative of a user's milk ejection reflex. The controller is configured to generate, in response to the input signal being received, an output for controlling pumping operation of the milk expression device to implement an expression pressure profile comprising a non-zero baseline under-pressure.

Also provided is a milk expression system comprising the controller and the milk expression device.

Further provided is a method and related computer program for controlling pumping operation of the milk expression device.

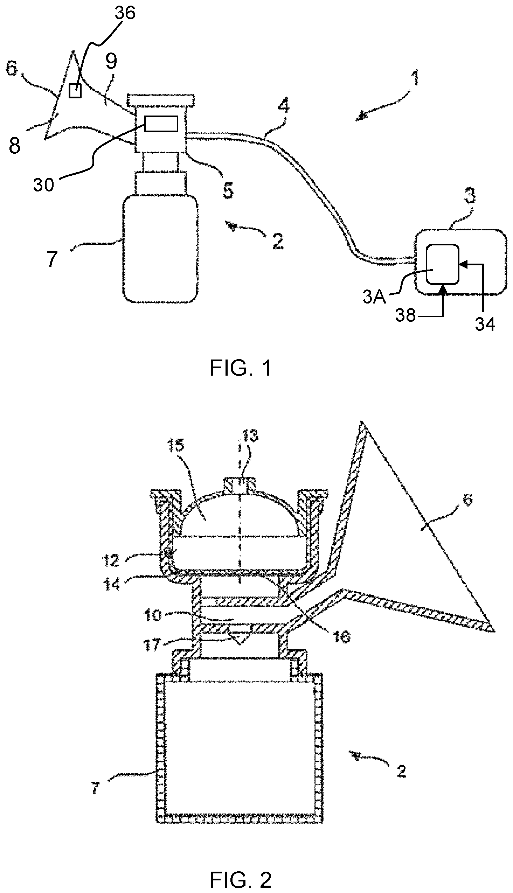

FIG. 1 shows a milk expression system 1 according to an example. The milk expression system 1 comprises an expression unit 2 and a milk expression device 3 whose pumping operation causes the expression unit 2 to expose a user's breast to a variable pressure.

The milk expression device 3 may be regarded as a breast pump. A controller 3A controls pumping operation of the milk expression device 3.

In some embodiments, such as shown in FIG. 1, the controller 3A is an integral component of the milk expression device 3, e.g. breast pump. Alternatively or additionally, the controller 3A may be included in a user device, for example a smart phone or tablet computer, separate from the milk expression device 3, and/or in a cloud-based server.

The expression unit 2 and the milk expression device 3 may be connected by a hose 4. The hose 4 can provide fluid communication between the expression unit 2 and the milk expression device 3.

The hose 4 may also be used to provide an electrical connection between the expression unit 2 and the milk expression device 3 and/or the controller 3A. For example, the hose 4 may supply an operating signal or electrical power between the expression unit 2 and the milk expression device 3 and/or the controller 3A.

In other embodiments, such as in the case of the milk expression system 1 being in the form of a wearable breast pump, the expression unit 2 is directly mounted and connected to the milk expression device 3.

Referring again to FIG. 1, the expression unit 2 may have a main body 5, a breast-receiving funnel 6 and a collection vessel 7 for collecting milk expressed from a user's breast. The collection vessel 7 may, for example, take the form of a feeding bottle, bag or any suitable container.

The collection vessel 7 may be attached to the main body 5 in any suitable manner. In some embodiments, such as shown in FIG. 1, the collection vessel 7 is releasably attached to the main body 5. The releasable attachment of the collection vessel 7 to the main body 5 is implemented by a screw fitting between the collection vessel 7 and the main body 5 in this particular non-limiting example.

The breast-receiving funnel 6, which is configured to receive a breast of the user, may extend from the main body 5.

The breast-receiving funnel 6 has a mouth 8 and a throat 9. The mouth 8 is open at an outer end of the breast-receiving funnel 6 to receive a user's breast, and the breast-receiving funnel 6 converges from the outer end towards the throat 9 to form a hollow recess in which a breast is receivable.

In such embodiments, the main body 5 may fluidly connect the breast-receiving funnel 6 to the collection vessel 7. In some embodiments, and referring now to FIG. 2, a fluid passageway 10 is formed through the main body 5 from the breast-receiving funnel's 6 hollow recess to the collection vessel 7.

In some embodiments, such as shown in FIGs. 1 and 2, the main body 5 is integrally formed with the breast-receiving funnel 6. In other embodiments, the main body 5 and the breast-receiving funnel 6 may be detachable from each other.

The main body 5 can be formed from any suitable material. In some embodiments, the main body 5 is formed from a plastic material, such as polypropylene.

In some embodiments, and referring to FIG. 2, a chamber 12 is formed in the main body 5, with the chamber 12 forming part of a vacuum path. In such embodiments, the chamber 12 may be in fluid communication with the fluid passageway 10 between the breast-receiving funnel 6 and the collection vessel 7.

The chamber 12 may have a vacuum port 13 for communicating with the milk expression device 3. In such embodiments, the hose 4 may be mountable to the vacuum port 13 to fluidly connect the chamber 12 with the milk expression device 3.

A membrane 14 may be received in the chamber 12. The membrane 14, also known as a diaphragm, is flexible. An outer rim of the membrane 14 may be mounted to the chamber 12.

The membrane 14 may be formed from any suitable flexible material. In some embodiments, the membrane 14 is formed from silicone.

The membrane 14 separates the chamber 12 into a first space 15 and a second space 16. The first space 15 communicates with the vacuum port 13. The first space 15 forms part of a first section of the vacuum path. The second space 16 communicates with the fluid passageway 10 between the breast-receiving space of the breast-receiving funnel 6 and the collection vessel 7. The second space 16 forms part of a first section of the vacuum path.

In at least some embodiments, such as shown in FIG. 2, a one-way valve 17 is disposed in the fluid passageway 10. Such a one-way valve 17 may prevent a pressure reduction being formed in the collection vessel 7. The one-way valve is, for example, a duckbill valve.

The flexible membrane 14 may have a predefined shape. In some embodiments, such as shown in FIG. 2, the membrane 14 has a substantially cup-shaped arrangement in a neutral condition, that is, when the membrane 14 is received in the chamber 12, but has not been deformed. However, it will be understood that the membrane 14 may have an alternative shape.

The milk expression device 3 may include a power source, a motor and a pump unit actuated by the motor. The pump unit may be configured to generate and release a pressure reduction, in other words vacuum, in the vacuum path, for example using a pressure relief valve separate to the pump unit, although these may be combined into a single unit. The controller 3A may control operation of these components of the milk expression device 3.

The controller 3A may operate the pump unit to implement a sequence of strokes, with each stroke comprising a vacuum increase phase and a vacuum release phase. During vacuum increase, the breast may be stimulated to express milk. This milk flows to the fluid passageway 10. During the vacuum release phase, the milk may pass through the one-way valve 17 into the collection vessel 7.

An opening is, for example, provided to allow air to escape from the collection vessel 7. This opening may be located, for instance, at the attachment, e.g. screw fitting, between the main body 5 and the collection vessel 7.

It is noted that the foregoing provides a description of only one example of a milk expression system 1. The present disclosure more generally relates to application and characteristics of pressure profile(s) applied during a breast pump session, and may be applied to milk expression systems 1 of any suitable design.

Referring again to FIG. 1, the milk expression system 1 comprises a sensor system 30 for generating an input signal indicative of a milk ejection reflex.

In some embodiments, the sensor system 30 comprises one or more of a milk delivery sensor for sensing milk delivery, a milk duct sensor for sensing a milk duct dimension, and a skin sensor for sensing one or more skin characteristics relevant to milk ejection reflex. Such sensors, and in particular their capability to generate an input signal indicative of a milk ejection reflex, will be described in more detail herein below.

More generally, the controller 3A comprises an input 34 for receiving an input signal, e.g. from the sensor system 30, indicative of the user's milk ejection reflex. The controller 3A is configured to generate, in response to the input signal being received, an output for controlling pumping operation of the milk expression device 3 to implement an expression pressure profile comprising a non-zero baseline under-pressure.

When a baby is being breast fed, and a milk ejection reflex occurs, the sucking rate of the baby is stimulating to increase milk intake. Vice versa, this higher sucking rate stimulates, in turn, milk release by the mother's abrupt emotional "drop". Hence a milk expression device 3, e.g. breast pump, maintaining a constant pulse-pump-rate may mean that actual milk collection and ejection are not optimized due to not being adapted to actual ejection from the user's milk ducts.

In the milk expression device 3, e.g. breast pump, a pump frequency parameter refers to how many times the milk expression device 3 sucks and releases per minute. A vacuum level parameter refers to the suction strength, or how strongly the milk expression device 3 sucks. "Smart" control over the milk expression device 3, in other words adaptation of such pressure-related parameter(s) to the user's milk ejection would be desirable.

The present invention is partly based on the insight that complete release of a nipple tissue area, due to an expression pressure profile employing a zero baseline under-pressure, upon a milk ejection reflex taking place provides sub-optimal mimicry of an infant's sucking behavior. This is because the infant tends not to completely release an under-pressure generated by their sucking when milk is being released. For this reason, a non-zero baseline under-pressure is employed in the expression pressure profile implemented via the controller's 3A control over the pumping operation of the milk expression device 3, e.g. breast pump, in response to the input signal indicative of the user's milk ejection reflex.

In other words, milk may mainly flow during periods in which there is a negative pressure, in other words under-pressure, not equal to zero. An issue with conventional milk expression device control is that the negative pressure varies between zero and a maximum level. While such a zero baseline under-pressure level might assist to increase milk quantity towards the end of the milk ejection reflex, completely releasing the nipple due to zero vacuum being applied, so as to relax the nipple tissue area, may not be optimal at earlier stages during the milk ejection reflex. The zero baseline under-pressure may contrast with a baby sucking and not releasing the vacuum applied to the nipple during milk release.

Hence the milk ejection reflex-responsive non-zero baseline under-pressure according to the present disclosure may provide enhanced mimicry of an infant's sucking behavior. In this way, milk extraction from the mother's breast using the milk expression device 3 may be enhanced.

In some embodiments, the non-zero baseline under-pressure, e.g. vacuum level, is maintained constant during a certain period of the milk ejection reflex, or can increase or decrease, e.g. linearly, such that minimum under-pressure/vacuum level in one pumping cycle is not equal to zero.

In some embodiments, pressure-related, e.g. vacuum level, parameters are adapted to milk delivery, e.g. milk flow velocity, not only by adjustment of frequency and maximum vacuum level but also by adjustment of the non-zero baseline vacuum level. Such embodiments will be described in more detail herein below with reference to non-limiting illustrative examples.

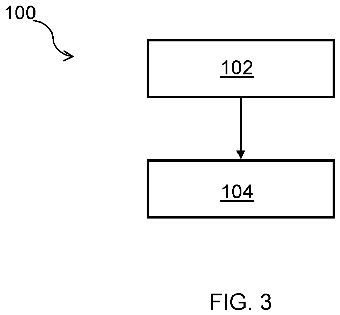

FIG. 3 provides a flowchart of a method 100 for controlling pumping operation of a milk expression device 3 according to any of the embodiments described herein. The method 100 comprises receiving 102 the input signal indicative of the milk ejection reflex, for example from the sensor system 30.

The method 100 further comprises generating 104, in response to the input signal being received, the output for controlling pumping operation of the milk expression device 3 to implement the expression pressure profile comprising the non-zero baseline under-pressure.

The method 100 may be implemented using the controller 3A according to any of the embodiments described herein.

In some embodiments, the method 100 comprises generating an initial output for controlling the milk expression device 3 to implement an initial pressure profile, such as a stimulation pressure profile.

It is noted that the stimulation pressure profile may stimulate the user of the milk expression device 3 to experience milk ejection reflex.

The initial output may, for example, be generated at the beginning of a session of use of the milk expression device 3, in other words breast pump session. The output for controlling pumping operation of the milk expression device 3 to implement the expression pressure profile comprising the non-zero baseline under-pressure may be generated 104 subsequently to the initial output being generated.

In some embodiments, when the input signal indicative of the milk ejection reflex is no longer received, for example due to milk flow falling below a predefined threshold, the method 100 may comprise controlling the milk expression device 3 to implement, e.g. revert to implementing, the initial pressure profile, in other words the pre-milk ejection reflex pressure profile.

The method 100 may comprise generating one or more intermediate outputs for controlling the milk expression device 3 to respectively implement the stimulation pressure profile at one or more intermediate stages during the session, e.g. between a first input signal indicative of milk ejection reflex being no longer received and a second input signal indicative of a subsequent milk ejection reflex being received.

This may reflect the fact that, during a breast pumping session, a milk flow pattern may evolve mainly due to the occurrence of milk ejection reflex. Thus, within one session, several milk ejection reflexes can occur.

In some embodiments, the method 100 comprises generating 104, in response to the input signal being received, an output signal for causing the non-zero baseline under-pressure to deepen compared to an initial baseline under-pressure provided prior to the input signal being received. In this way, milk flow may be promoted following onset of the milk ejection reflex. It is noted that the initial baseline under-pressure may, for example, be included in the above-mentioned stimulation pressure profile.

Alternatively or additionally, the generating 104 the output, in response to the input signal being received, comprises generating 104 output signal data for causing the non-zero baseline under-pressure to be maintained during a main period during which the expression pressure profile is implemented, and then to diminish during an end period that follows the main period. By the non-zero baseline under-pressure diminishing during the end period relative to in the main period, the change in the baseline under-pressure may reflect decreasing milk flow that occurs as the milk ejection reflex is ending.

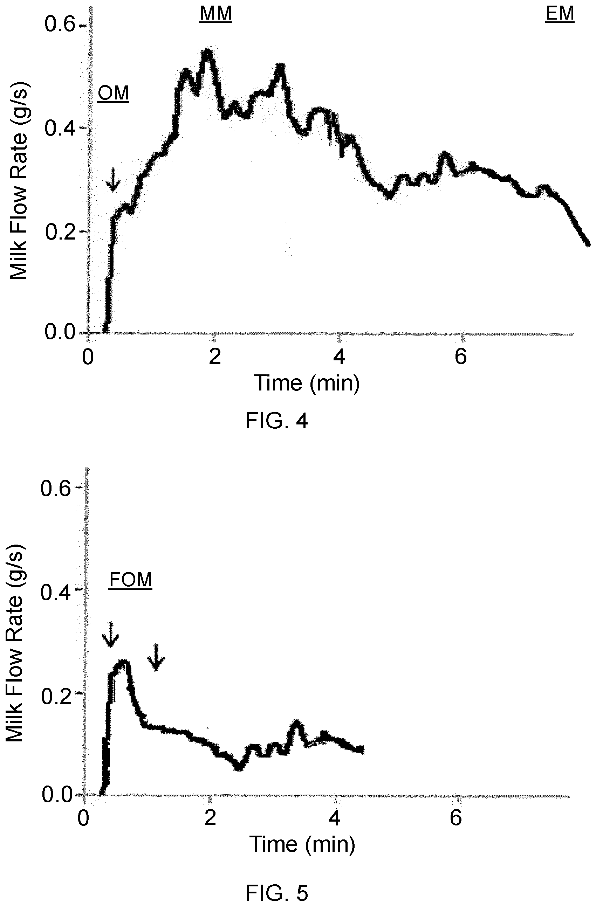

FIG. 4 provides an impression of expected results from a milk expression device 3 being controlled in accordance with the present disclosure. As soon as the milk ejection reflex onset OM is indicated, e.g. detected via milk flow, the pressure-related/pumping parameters are adapted to, e.g. optimize for, natural milk ejection reflex and milk flow.

For example, pressure differences between maximum and baseline under-pressures may be reduced to milk ejection reflex-maintaining levels, as well as the non-zero baseline under-pressure being maintained, e.g. on a level designed for a specific phase of the milk ejection reflex.

It is evident from FIG. 4 that milk flow may be relatively high and relatively stable during a main phase of the milk ejection reflex MM, e.g. in comparison to the above-mentioned conventional milk expression device control in which the negative pressure varies between zero and a maximum level. The end phase of the milk ejection reflex is denoted in FIG. 4 by EM.

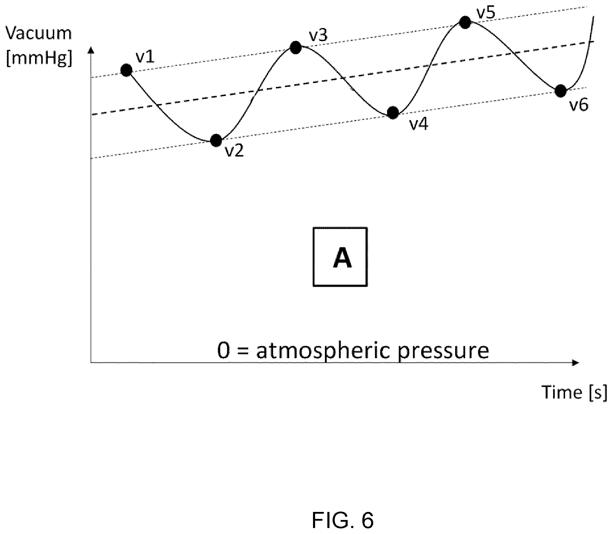

FIGs. 6 to 8 graphically show pressure-related parameters, e.g. vacuum parameters, for different periods during which the milk expression profile is implemented. These periods can be regarded as different phases of the milk ejection reflex, noting that "v" corresponds to a measured flow velocity of the milk.

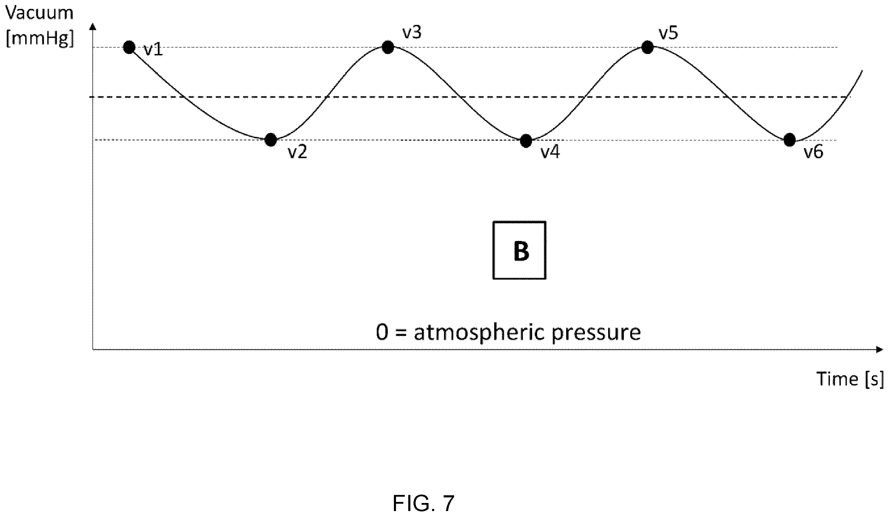

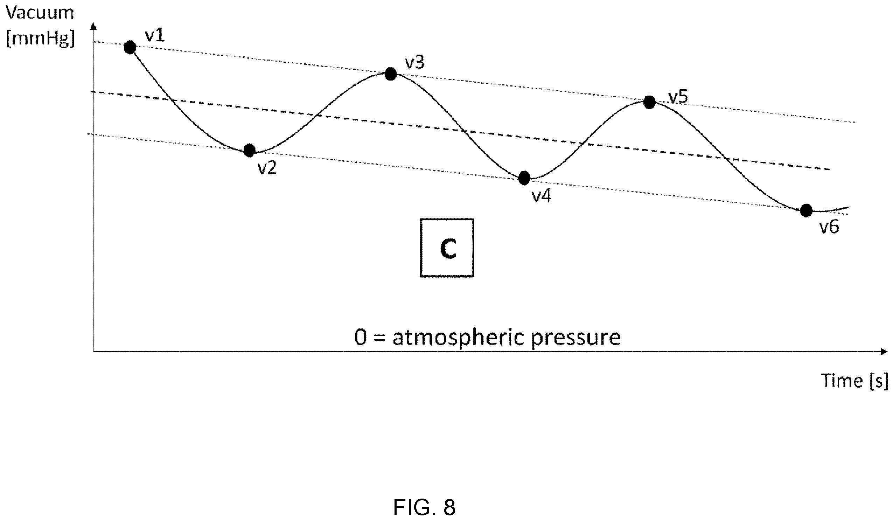

In FIG. 6, the measured flow velocity v3 is higher than v 1, and v5 is greater than v3, etc. The graph "A" provided in FIG. 6 could correspond to the milk expression profile employed during onset of milk ejection reflex OM, the graph "B" provided in FIG. 7 could correspond to the milk expression profile employed during the main phase of the milk ejection reflex MM, and the graph "C" provided in FIG. 8 could correspond to the milk expression profile employed during the end phase of the milk ejection reflex EM.

Immediately following milk ejection reflex onset OM, milk flow may start to increase and the pressure-related parameter(s) are preferably adjusted accordingly, in particular by the baseline under-pressure deepening, as shown in FIG. 6.

The cycle frequency may also be decreased with respect to the initial pressure profile, e.g. the stimulation pressure profile.

During the main phase of the milk ejection reflex MM, the non-zero baseline under-pressure may be maintained constant, as shown in FIG. 7.

After the main phase of the milk ejection reflex MM, proceeding to the end-of-milk ejection reflex phase EM, milk flow may start to decrease and pressure-related parameters are preferably adjusted accordingly, in particular by the non-zero baseline under-pressure diminishing, as shown in FIG. 8. The pressure-related parameters may, for example, revert to those employed in the initial pressure profile, e.g. stimulation pressure profile, following the end phase of the milk ejection reflex EM.

At this point it is noted that an issue with certain conventional ways of controlling milk expression devices 3 is that switching of pressure-related parameters so as to implement a milk expression profile may be falsely triggered, e.g. with consequential interruption of the stimulation mode prolonging the time it takes to (re-)stimulate the user.

A false onset milk ejection reflex FOM is shown in FIG. 5. It is evident from FIG. 5 that during the false onset milk ejection reflex FOM a relatively prolonged decrease in milk flow takes place.

For this reason, the generating 104 of the output may be in response to the input signal being indicative of the milk ejection reflex being maintained for at least a predetermined duration. This may assist to minimize the risk of false identification of the milk ejection reflex because the predetermined duration may allow time for the above-mentioned decrease in milk flow to be identified.

It is noted that the decrease in milk flow may be triggered by the pre-milk ejection reflex pressure profile/pumping cycle. Hence it may be beneficial to take steps to minimize or prevent triggering of such a false onset milk ejection reflex FOM by pumping of the milk expression device 3.

To this end, the method 100 may comprise generating, during the predetermined duration, a control output for controlling pumping operation of the milk expression device 3 to implement an adaptation pressure profile in which one or more pressure-related parameters are progressively adjusted towards the expression pressure profile comprising the non-zero baseline under-pressure.

Thus, the milk expression device 3 may continue to pump during suspected milk ejection reflex onset OM and adopt parameter(s) towards the expression pressure profile. Such parameters, and in particular the non-zero baseline under-pressure, may be adapted for established milk ejection reflex.

This control over the milk expression device 3 may compensate for possible influence of the milk expression device's 3 pumping on development of the milk ejection reflex. For example, the milk expression device 3 may be controlled to continue pumping during milk ejection reflex onset OM, in other words during the predetermined duration, and gradually adopt pressure-related parameters towards the main phase of the milk ejection reflex MM, e.g. towards the above-mentioned non-zero baseline under-pressure that is deepened relative to the initial baseline under-pressure.

This may be superior to updating the pressure-related/pumping parameters when the suspected milk ejection reflex onset OM is detected but immediately resuming the stimulation pressure profile upon identification of the false onset milk ejection reflex FOM. This is because the progressive adjustment towards the expression pressure profile may assist to minimize the risk that changes to the pressure-related/pumping parameters, e.g. and associated disturbance of pump rhythm, negatively influence stimulus received by the breast.

In some embodiments, the method 100 comprises generating, for example reverting to generate, the initial output for controlling the milk expression device 3 to implement the initial pressure profile in response to the input signal indicative of the milk ejection reflex not being maintained for the predetermined duration.

In such embodiments, the method 100 may comprise reverting to generate the stimulation pressure profile in response to the input signal indicative of the milk ejection reflex not being maintained for the predetermined duration.

Thus, the method 100 may enable relatively straightforward and timely detection of false milk ejection reflex onset FOM, and return to, for instance, normal pre-milk ejection reflex pressure-related/pumping parameters.

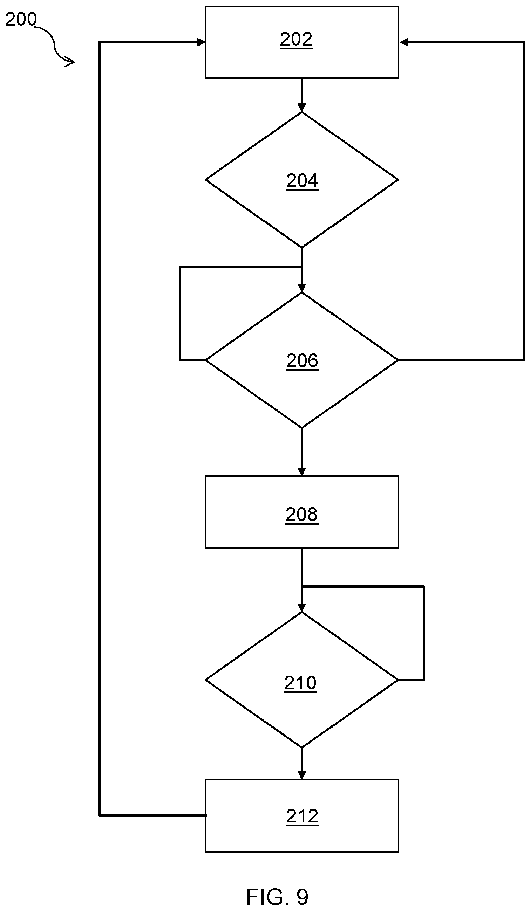

FIG. 9 provides a flowchart of a method 200 according to an illustrative non-limiting example. The method 200 starts with a step 202 comprising generating an initial output for controlling the milk expression device 3 to implement the initial pressure profile, such as the stimulation pressure profile. The initial pressure profile may, for example, be implemented via pumping parameters and stimulus configured, e.g. optimized, for pre-milk ejection reflex.

Step 202 may further comprise monitoring a signal, e.g. received via the input 34. Monitoring this signal may, for example, enable monitoring of milk flow.

Step 204 corresponds to detection of a suspected/possible milk ejection reflex, for instance detected by monitoring milk flow. This step 204 may correspond to receiving 102 the input signal indicative of the milk ejection reflex.

In some embodiments, the suspected milk ejection reflex may be detected 204 by milk flow being a above a minimum threshold.

Step 206 corresponds to monitoring the input signal indicative of the milk ejection reflex to determine whether the input signal is maintained for at least a predetermined duration. This may comprise, for example, monitoring milk flow to determine whether the increased milk flow indicative of the milk ejection reflex is maintained, e.g. during the milk ejection reflex onset OM.

Step 206 may be implemented repeatedly during the milk ejection reflex onset OM. Should the input signal indicative of the milk ejection reflex not be maintained for the predetermined duration, the method 200 reverts to step 202. For example, should milk flow decrease over a relatively prolonged period, the milk ejection reflex onset OM is determined to be a false milk ejection reflex onset FOM, and the pre-milk ejection reflex pumping and stimulus parameters may be reverted to or maintained at step 202.

As described above, the signal monitoring 206, for example monitoring of the increased milk flow, may be accompanied by compensation for possible influence of the milk expression device's 3 pumping, e.g. pumping cycle influences, on milk ejection reflex onset OM.

Turning to step 208, the milk ejection reflex may be optimized by tuning pressure-related parameter(s) and vacuum, as described above in relation to FIGs. 6 to 8. Thus, the milk flow may be maintained or further increased should a real milk ejection reflex onset OM be detected and the parameters can be tuned to optimize the milk ejection reflex.

In other words, during the main milk ejection reflex phase MM, the pressure-related parameters could be tuned, e.g. to follow the milk ejection reflex according to how the milk ejection reflex evolves.

Step 210 corresponds to repeatedly monitoring the input signal to detect the end of the milk ejection reflex, at which point the method 200 reverts at step 212 back to step 202. For example, when the milk ejection reflex ends, the milk flow may reach or fall below a specific threshold and the milk expression device 3, the pressure-related and/or stimulus parameters, etc. could return to their initial, pre-milk ejection reflex state at step 212.

As soon as a possible milk ejection reflex onset OM is detected, for example, via the milk flow and/or smart classifiers, the milk flow should increase during main milk ejection reflex MM, referring again to FIG. 4, if the detected milk ejection reflex is real/genuine.

The present disclosure provides an algorithm that activates adaptation of pressure-related/pumping parameters by recognizing milk ejection reflex onset OM, in other words the start of milk ejection reflex, e.g. based on milk flow and/or smart classifiers, such as tissue perfusion, systemic measures, etc., as described in more detail herein below. Such smart classifiers may be unique and personalized to the user.

More generally, any suitable input signal can be used to trigger the expression pressure profile comprising the non-zero baseline under-pressure, provided that the input signal is indicative of the milk ejection reflex.

In some embodiments, the sensor system 30 comprises a sensor configured to sense milk flow parameter(s), such as an acoustic sensor, relating to the milk ejection reflex, and/or combinations of parameters, e.g. flow-related parameter.

The input signal may therefore derive from a flow sensor, e.g. an optical flow sensor, integrated in the expression unit 2 and/or the milk expression device 3. Such a flow sensor may sense, for instance, milk flow velocity. The flow sensor can, for example, be integrated in the breast-receiving funnel 6.

Alternatively or additionally, the milk flow velocity may be directly measured noninvasively in the duct area by making use of an optical system, such as a laser doppler, laser speckle, doppler Optical Coherence Tomography (OCT), and/or photoplethysmography (PPG) system integrated in the milk expression device 3 and/or the expression unit 2. In such embodiments, the optical system may include, for example, a VIS/NIR laser, light emitting diode or super luminescent diode (SLD) for deep penetration at the nipple area.

Alternatively or additionally, a non-optical unit may measure skin parameters, such as skin perfusion, skin temperature, skin impedance, and/or skin deformation.

Classifiers may be used to predict optimum milk flow parameters for the specific state of milk ejection reflex. For example, a duct diameter and/or a duct dilation may be measured with a single-photon avalanche diode (SPAD) sensor, as will be described in more detail herein below.

In some embodiments, the generating 104 the output for controlling pumping operation of the milk expression device 3 to implement the expression pressure profile comprising the non-zero baseline under-pressure is in response to a milk delivery signal relevant to milk delivery from the user's breast.

In such embodiments, the milk delivery signal may be relevant to a milk flow and/or a milk amount from the user's breast.

The milk delivery signal may provide a relatively straightforwardly implementable way of detecting the milk ejection reflex. To this end, the sensor system 30 that generates the milk delivery signal may include a milk delivery sensor, for example an optical milk delivery sensor and/or an acoustic milk delivery sensor, for sensing milk delivery.

The optical milk delivery sensor may operate using any suitable detection principle. For example, the optical milk delivery sensor may employ laser doppler, laser speckle, doppler optical coherence tomography (OCT), photoplethysmography (PPG), visible/near-infrared laser, LED and/or super luminescent diode (SLD) technology.

In some embodiments, the sensing is based on detecting interruption of a light beam. Alternatively, it may be based on measurement of a change in reflection or scattering of light back from a layer of milk.

The acoustic milk delivery sensor may, for example, include a microphone for detecting a sound indicative of milk transport and/or collection.

In some embodiments, the method 100 comprises generating 104 a milk delivery-based control signal for setting a level of the non-zero baseline under-pressure based on the milk delivery signal. Thus, the non-zero baseline under-pressure can be aligned with milk delivery, for instance such that a deeper non-zero baseline under-pressure is employed when milk delivery is higher and/or increasing, and a diminished non-zero baseline under-pressure is employed when milk delivery is lower and/or decreasing.

As an alternative or in addition to the milk delivery signal, the input signal may comprise a skin characteristic signal.

To this end, the sensor system 30 that generates the skin characteristic signal may include a skin sensor for sensing one or more skin characteristics relevant to milk ejection reflex, such as a skin perfusion, skin temperature, skin impedance, and/or skin deformation. Such a skin sensor may include an optical skin sensor and/or a non-optical skin sensor.

In some embodiments, the method 100 comprises generating 104 the output in response to a milk duct dimension signal relevant to a milk duct dimension. In such embodiments, milk duct dilation that occurs with onset of the milk ejection reflex may be utilized in controlling the pumping operation of the milk expression device 3 to implement the expression pressure profile.

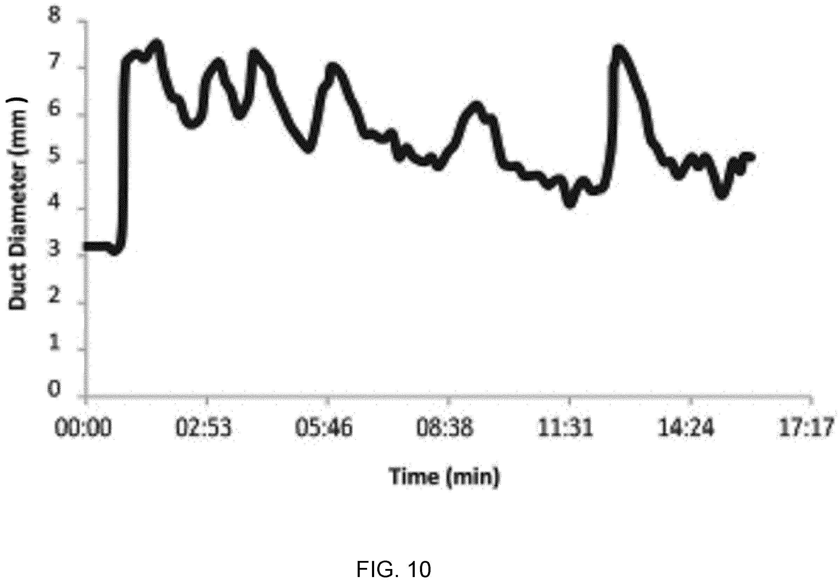

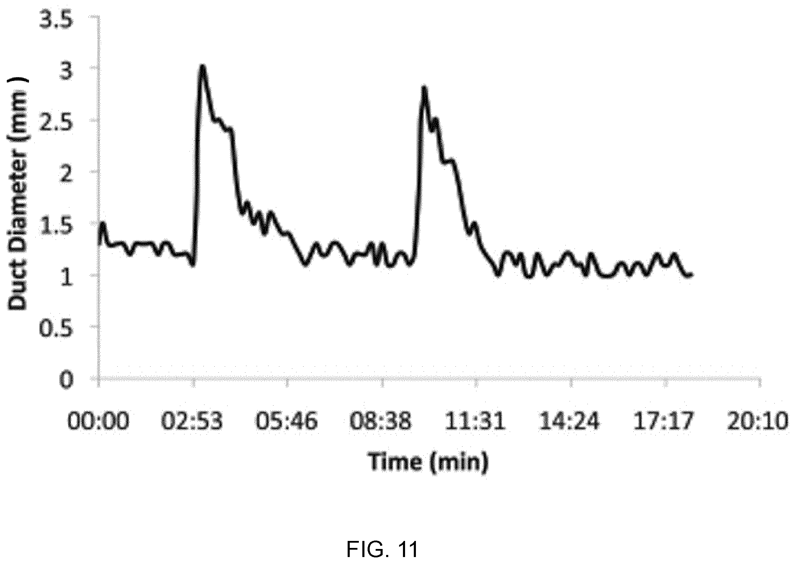

Quantifying the milk ejection reflex to enhance the breastfeeding experience and give guidance concerning milk supply may represent a key challenge in the context of breastfeeding. If the mother has blocked milk ducts, locating blocked regions can help to provide early mastitis prevention. In the case of milk expression/breast pumping, conventional milk expression devices 3 may not be adaptable to the milk ejection reflex of each individual mother. The graphs provided in FIGs. 10 and 11 illustrate different milk ejection reflexes associated with different duct dilations in two different women (see Geddes D. T., International Breastfeeding Journal, 2009, 4, 1-7). It is correspondingly desirable to objectively measure milk flow. Milk flow may be directly correlated with lactiferous duct dilation during the milk ejection reflex.

By quantifying lactiferous duct dilation during the milk ejection reflex, the milk expression device 3, e.g. breast pump, can be controlled/adapted for specific milk ejection per woman and session. For breastfeeding, duct dilation quantification can also give guidance on perceived milk supply issues and milk duct blockages.

Moreover, using milk flow to identify the milk ejection reflex can, in certain cases, lead to a false milk ejection reflex onset FOM event, as described above. Hence, the present disclosure proposes use of lactation/milk duct dilation change to detect milk ejection reflex. In particular, the present disclosure partly concerns quantifying lactiferous duct dilation during the milk ejection reflex.

FIG. 11, for example, illustrates milk duct diameter change, due to dilation, with onset of the milk ejection reflex. Adaption of pressure-related parameters/pump parameters can be closely linked to the onset of the duct diameter changes. Furthermore, duct dilation can be used to identify blocked regions in the breast. In such embodiments, the vacuum level can, for instance, be further adjusted to alleviate pain and/or enhance comfort should duct(s) be blocked. For example, the vacuum level can be adapted so as to avoid creating an overly strong suction force on a breast with a blocked duct.

In some embodiments, the generating 104 the output is in response to the milk duct dimension signal being indicative of a milk duct dimension parameter reaching or passing a given threshold.

Alternatively or additionally, the method 100 may comprise generating 104 a duct dimension-based control signal to set a magnitude of the non-zero baseline under-pressure based on the milk duct dimension signal. The milk duct dimension parameter may, for example, be a milk duct dimension and/or a change, e.g. increase, in a milk duct dimension.

In some embodiments, and referring to FIGs. 1, 12 and 13, the sensor system 30 generates the milk duct dimension signal by including a milk duct sensor, for example an optical milk duct sensor, for sensing milk duct dimensions.

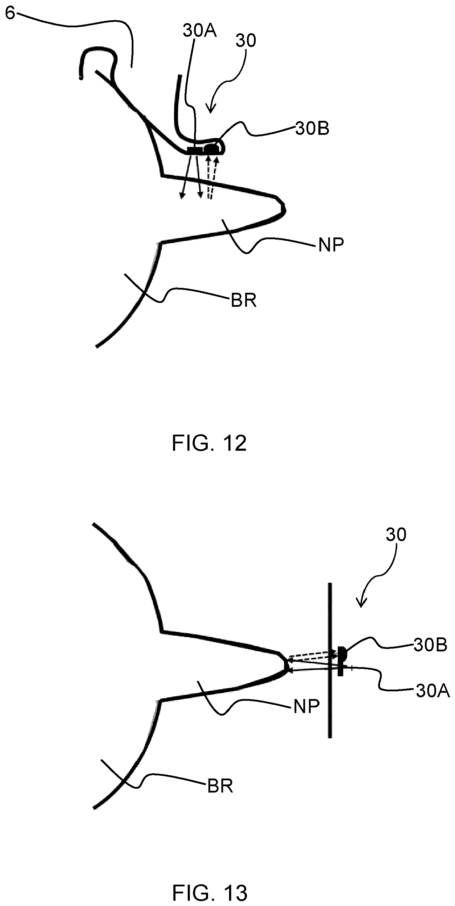

In such embodiments, at least part of the milk duct sensor may be included in the expression unit 2. Examples of this are shown in FIGs. 12 and 13.

In particular, at least part of the milk duct sensor may be mounted on the breast-receiving funnel 6, as shown in FIG. 12.

In general terms, the milk duct sensor can be applied in a nipple shield in the case of milk duct dilation monitoring during breastfeeding, or in a wearable\manual\electric breast pump 3 for milk duct dilation monitoring during breast pumping.

In some embodiments, the optical milk duct sensor includes a light source 30A, e.g. a laser, arranged to direct light onto the breast BR, for example onto the nipple NP. The optical milk duct sensor may also include an optical detector 30B, e.g. a camera, arranged to receive light from the breast BR, for example from the nipple NP.

In some embodiments, such as shown in FIG. 12, the optical milk duct sensor is arranged at a side of the nipple NP, and as such light from the light source 30A may be directed from this side and the optical detector 30B may be arranged at the side to receive light reflected from the nipple NP.

In other embodiments, such as shown in FIG. 13, the optical milk duct sensor is arranged at a front of the nipple NP, and as such light from the light source 30A may be directed from in front of the nipple NP and the optical detector 30B may also be arranged in front of the nipple NP.

The optical milk duct sensor may operate using any suitable detection principle. In some embodiments, the optical milk duct sensor comprises a single-photon avalanche diode (SPAD) sensor.

The relatively high speed acquisition of such an optical sensor may enable quickly occurring milk ejection reflex events to be identified. This may be due to the highspeed photon counting achievable with a SPAD sensor.

SPAD sensors measure each individual light particle, in other words photon, that reaches the pixel. Each photon that enters the pixel immediately gets converted into an electric charge, and the electrons that result are eventually multiplied like an avalanche until they form a large signal charge that can be extracted. SPAD sensors allow recording of the arrival times of the photons providing nanosecond range time domain histograms for each pixel.

SPAD sensors may have various advantages over, for example, conventional CMOS image sensors and OCT-doppler flow imaging sensors. CMOS sensors may give intensity values and provide a two-dimensional image from the intensity values, whereas a SPAD sensor may provide a two-dimensional image, a three-dimensional depth map, from direct time of flight, and a photon arrival time histogram per pixel. SPAD sensors may also benefit from having a potentially less complicated optical design, e.g. using relatively small laser diode(s) 30A and a SPAD detector 30B.

In some embodiments, the optical milk duct detection comprises photon counting with time-domain visible, near-infrared spectroscopy.

This may provide reliable, non-invasive milk duct diameter change detection, which is correlated with milk ejection reflex, inside the breast tissue.

It is noted that time-domain near-infrared spectroscopy has already been implemented in blood flow measurement for brain function, referring to Ban et al., Journal of Biomedical Optics, 2022, 27,074710.

In some embodiments, the optical milk duct sensor comprises a SPAD sensor in combination with time-domain near-infrared spectroscopy in at least three wavelength ranges, preferably 450-550 nm, 900-950 nm, and 950-1000 nm.

In such embodiments, the optical milk duct sensor may comprise a camera image sensor 30B and a plurality of lasers 30A for providing the at least three wavelength ranges.

Such an optical milk duct sensor may discriminate milk from surrounding tissue based on lipid, water and beta carotene. The three wavelengths may enable water/interstitial fluid, fatty tissue/beta carotene and milk, in other words fatty liquid, to be differentiated from each other.

During milk flow resulting from the milk ejection reflex, in both breast feeding and milk expression/breast pumping, it is emphasized that there may be a correlation between breast duct dilation and milk flow. Absorption change, e.g. change in time distribution profile of photon arrival times per wavelength using SPAD sensing, in the three wavelength ranges, respectively associated with water, lipid and beta carotene, can then be used to detect the milk duct dilation.

The 900-950 nm wavelength range may reflect that a fat/lipid absorption peak in a tissue absorption window is present in this wavelength range. The effective penetration depth in this wavelength range may be several millimeters. This is sufficient as the depth distribution of milk ducts may be 1 to 8 mm, referring to, for example, Rusby et al., Breast cancer research and treatment, 2007, 106, 171-179.

The 450-550 nm wavelength range may reflect that an absorption peak from riboflavin and beta carotene present in milk is present in this wavelength range, referring to, for instance, Stocker et al., Applied spectroscopy, 2017, 71, 951-962; Bosschaart et al., Journal of biomedical optics, 2019, 24, 056006; and de Boer, Breast Cancer Res. Treat., 2015, 152:509-518.

The penetration depth in this 450-550 nm wavelength range may be less than 1 mm. Hence this wavelength range may only be used for superficial milk ducts on the breast tissue. For the use-case of adapting the pressure-related/pumping parameters of a milk expression device 3, e.g. a wearable breast pump, using detected milk flow, superficial milk duct flow would be sufficient. Additionally, superficial ducts dilate and expand during milk ejection making the less than 1 mm depth sufficient to observe superficial ducts.

The 950-1000 nm wavelength range reflects that a water absorption peak in the tissue absorption window is present in this wavelength range. A similar water absorption peak can be observed for milk. The effective penetration depth in this wavelength range may be several millimeters.

In addition to the wavelength ranges employed in the time-domain near-infrared spectroscopy, use may be made of concentrations, e.g. percentages, of lipids, water, and beta carotene in surrounding tissue types, such as nonlactating breast tissue, referring to, for example, Nachabe, et al., Journal of biomedical optics, 2011, 16, 087010. Spatiotemporal concentration changes in mammary tissue for a lactating breast in the first 237 days from giving birth have also been observed, referring again to Bosschaart et al., Journal of biomedical optics, 2019, 24, 056006. Water concentration may be lower in surrounding tissue types compared to the nipple area, due to the latter being filled with milk ducts.

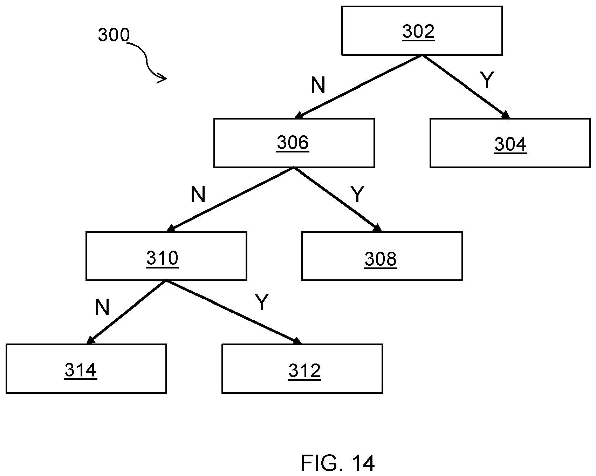

FIG. 14 shows a simple decision tree algorithm 300 that can be used to discriminate between contributions from water, lipid and beta carotene and identify if breast tissue or milk is being measured. Since lipid content in milk is about 2-4%, any lipid content measured in the 900-950 nm wavelength range that is higher than 20% may indicate tissue and not milk. A measured beta carotene content of more than 1 µM in the 450-550 nm wavelength range may be indicative of tissue, since the concentration of beta carotene in milk may be about 33 nM, referring to Xavier et al., Antioxidants, 2019, 8, 291. A measured water content of less than 60%, using the 950-1000 nm wavelength range, may be indicative of milk, since the water content in milk is greater than 85%.

In this non-limiting example, a laser diode 30A illuminates the lactating breast with the above-mentioned 900-950 nm, 450-550 nm and 950-1000 wavelengths time domain multiplexed in pulse windows in range of picoseconds. A SPAD sensor 30B captures a distribution (histogram) of time of flight photons reflected from the breast tissue for each wavelength. The SPAD sensor 30B provides a histogram of photon arrival times for a sampling window. A typical SPAD sensor can resolve up to 6 ns in each histogram bin, referring again to Ban et al., Journal of Biomedical Optics, 2022, 27, 074710.

This distribution of photon arrival can be converted to optical properties of a material using the moments of distributions of time of flight of photons, referring to, for example, Liebert, et al., Applied optics, 2003, 42, 5785-5792. This may provide the absorption coefficient of the material.

Given the expected molar absorption coefficient of material, this absorption coefficient can be converted to a concentration value of the material in the breast tissue using the modified Beer-Lambert law.

The decision tree algorithm 300 may be used to select and filter, from all the pixels in the SPAD, only the pixels that are observing a milk duct.

In step 302, it is assessed whether lipid concentration is greater than 10%. If yes, tissue is identified at 304. If no, it is assessed whether beta carotene concentration is greater than 1 µM in step 306. If yes, tissue is identified at 308. If no, it is assessed whether water concentration is less than 60% in step 310. If yes, tissue is identified at 312. If no, milk is identified at 314.

For those selected pixels and using the wavelength range for water, the above computation may be repeated for the next sampling window and concentration of milk may be computed for that new time window. With this, a time-varying milk concentration signal can be computed. This is the milk duct dimension signal, in other words the milk duct dilation signal.

Given the duct dilation during milk ejection, higher absorption of milk can be expected after dilation. Therefore, a change in distribution of arrival times of photons is expected and an associated concentration change. With this change, the time-varying milk concentration can be used to determine milk ejection metrics.

In some embodiments, the histogram of photon arrivals is used to compute dilation and detect milk ejection reflex events. A shift of the histogram can be expected over time as depth of the milk duct changes due to dilation. Given that time distribution of arrival times of photons may correlate with tissue depth, this depth can be computed from the peak of the histogram. This shift may vary over time and this time-varying shift can be used to generate the milk duct dimension signal, e.g. of the type shown in FIGs. 10 and 11.

In some embodiments, time varying effects in the lipid wavelengths are used to compute milk concentration changes and to compute a time varying signal, as above, but with the signal containing time varying effects of oxyhaemoglobin and deoxyhaemoglobin.

In such embodiments, an additional laser with a 825-850 nm wavelength range can be used to obtain absorption values of oxyhaemoglobin and deoxyhaemoglobin, as the absorption coefficients are similar to the 900-950 nm case described above.

The oxyhaemoglobin and deoxyhaemoglobin absorption coefficients can be used for normalization of the measured values for lipid/fat. Additionally, time distribution of arrival times of photons for oxyhaemoglobin and deoxyhaemoglobin can be used to normalize time distribution of arrival times of photons for lipid.

In some embodiments, prior anatomical knowledge relevant to lactation, for example depth of lipid, milk duct depth, can be used filter out sections of the distribution of arrival times of photons for a wavelength. Given that time distribution of arrival times of photons may correlate with tissue depth, this depth can be computed from the peak of the histogram. This time value, and the associated depth, can be used to filter out the distribution of arrival times of photons for a given wavelength.

The peak of the histogram in the 900-1000 nm water wavelength range around the expected milk duct depth can confirm presence of milk ducts. Similarly, the peak of the histogram in the 900-950 nm lipid wavelength range around the expected depth of adipose tissue can confirm presence of adipose.

Due to duct stretches caused by pumping by the milk expression device 3, assessment of duct dilation may be performed at a defined/fixed position of the nipple NP, e.g. in the expression unit 2, such as in the breast-receiving funnel 6.

This defined position is ideally synchronized with the pumping frequency of the milk expression device 3, e.g. breast pump.

In some embodiments, multiple combinations of source 30A and detector 30B systems can be implemented to cover the complete breast.

In such embodiments, multiple time-varying milk duct dimension signals can be computed for different spatial regions in the breast tissue. With multiple signals, lack of dilation, due to milk duct blockages, in one signal can be spatially correlated to find regions with milk duct blockages.

In some embodiments, time-varying milk duct dilation signals over multiple feeding or pumping sessions can be correlated to enable milk supply changes to be observed, e.g. over relatively long time durations, such as about 6 months.

It is noted that causes of lactiferous duct blockages are failure to remove milk from part of the breast or insufficient emptying of the breast. In the context of breast feeding, insufficient emptying of the breast may be due to the baby not feeding. In the context of milk expressing, insufficient emptying of the breast may be due to an insufficient pumping time or a suboptimal vacuum profile being employed during milk expression.

Lactiferous duct blockages can lead to further complications to the mother. Mastitis is one common complication due to milk duct blockages. Currently, the mother tends to apply warmth alone or in combination with manually massaging the breast to drain the milk and clear the blockage.

In some embodiments, the method 100 may include adjusting the expression pressure profile according to a duct blockage level, for example fully blocked or partially blocked. Equivalently, the controller 3A may be configured to adjust the expression pressure profile according to a duct blockage level, for example fully blocked or partially blocked.

This adjustment of the expression pressure profile may, for example, be based on the milk duct dimension signal and/or on one or more parameters of the expression pressure profile.

The controller 3A may, for instance, be configured to implement a milk drain mode that adapts the milk expression device 3, e.g. smart pump, to alleviate specific duct blockages, and/or provide live adaption to assist with milk duct blockage reduction.

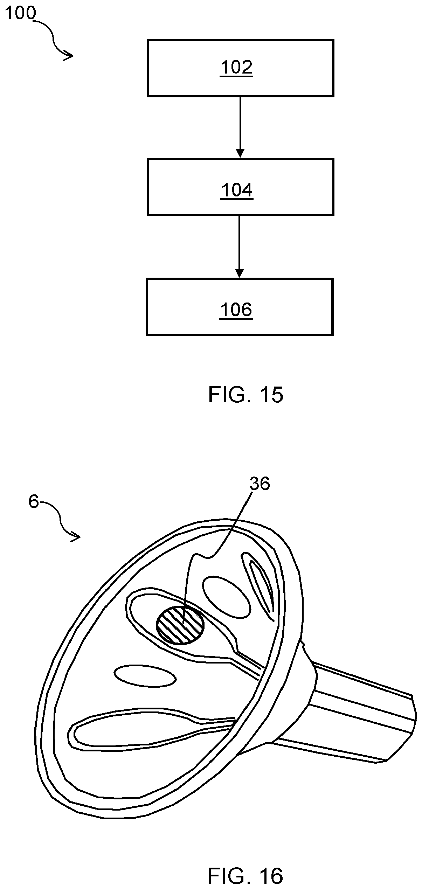

In some embodiments, and referring to FIGs. 15 and 16, the method 100 comprises generating 106, in response to the input signal, a further output for controlling one or more mechanical vibration units 36 for vibrating at least part of the user's breast. This may assist to alleviate blocked duct(s), with the vibration of the vibration unit(s) 36 operating in tandem with the non-zero baseline under-pressure to assist with unblocking duct(s).

It is noted that the ordering of steps shown in FIG. 15 should not be regarded as being limiting and in some embodiments the generating 106 of the further output for controlling the vibration unit(s) 36 is implemented simultaneously or prior to the generating 104 of the output for controlling pumping operation of the milk expression device 3 to implement the expression pressure profile comprising the non-zero baseline under-pressure.

In some embodiments, the further output for controlling the mechanical vibration unit(s) 36 is based on the milk duct dimension signal and/or on one or more parameters of the expression pressure profile.

The vibration unit(s) 36 may be activated, e.g. by the controller 3A, for regions of the breast determined to include blocked ducts.

Alternatively or additionally, the controller may be configured to control the mechanical vibration unit(s) 36 to vibrate, e.g. massage, the breast when the expression pressure profile is being implemented. In this manner, milk in the blocked ducts can be suctioned and drained out.

In some embodiments, the method 100 may comprise adapting vibrating provided by the vibration unit(s) 36 based on the milk duct dimension signal, for example as a diameter of a blocked duct changes.

Such adaptation of the vibrating, e.g. vibrating level and/or vibrating frequency, provided by the vibration unit(s) 36 can be implemented in real time, for example together with adjustment of parameter(s) of the expression pressure profile.

Thus, the milk expression device 3 may be controlled to operate in the milk drain mode that is used for clearing milk duct blockages. A first step may be to identify/compute regions of the breast that contain milk duct blockages and/or assess blockage level, e.g. fully blocked or partially blocked. This can be based on optical detection, such as the SPAD sensing described above.

In the drain mode, the vibration unit(s) 36 may activated for the identified/computed region(s) of the breast. A vacuum level may be delivered by the milk expression device 3 that is linked to the blockage level.

Alternatively, massage can start at a certain moment, for example during milk ejection reflex onset, when a certain vacuum level is applied. With that, milk in the blocked ducts can be suctioned and drained out. With breast massage using vibration unit(s) 36, as the duct diameter of a blocked duct changes, parameter(s) can be adapted in real-time, for example via adjustment of vibrating level, vibrating frequency and/or vacuum level.

In some embodiments, and referring specifically to FIG. 16, the vibration unit(s) 36 is, or are each, contained in a groove of the breast-receiving funnel 6, e.g. a silicone flange thereof.

In such embodiments, the breast-receiving funnel 6, e.g. silicone flange, may include multiple grooves that each contain a vibration unit 36.

This may provide massaging of the breast akin to that provided by a massage gun.

In some embodiments, and referring again to FIG. 1, the controller 3A comprises a further input 38 for receiving a further input signal indicative of a maximum milk flow and/or a degree of discomfort experienced by the user. In such embodiments, the controller 3A may be configured to generate a control signal to set an upper level of the under-pressure in the expression pressure profile based on the further input signal.

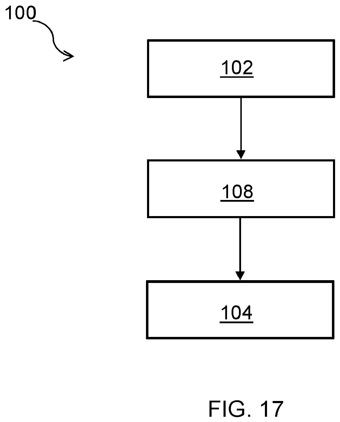

Equivalently, and referring to FIG. 17, the method 100 may comprise receiving 108 the further input signal indicative of a maximum milk flow and/or a degree of discomfort experienced by the user. The generating 104 the output for controlling pumping operation of the milk expression device 3 to implement the expression pressure profile comprising the non-zero baseline under-pressure may include setting an upper level of the under-pressure in the expression pressure profile based on the further input signal.

This may assist to make milk expression using the milk expression device 3 more comfortable for the user.

The upper level of the under-pressure, e.g. upper vacuum threshold, may be set as boundary condition by user input discomfort or pain level, for example as determined by pain response sensors, e.g. integrated into the expression unit 2, such as integrated into the breast-receiving funnel 6, and/or by maximum milk flow.

Such pain response sensors can be part of the system to keep parameter(s), such as the upper level of the under-pressure, between boundaries of the comfort and pain level.

Maximum and/or minimum vacuum levels can be set by the user, either before use, or as a satisfaction parameter after the pumping session. Moreover, maximum milk flow can be set as boundary condition.

It is noted that an objective measure of a pain can be acquired based on biosignal(s), such as from a skin sensor, e.g. a skin conductance sensor, a heart rate sensor and/or any other sensor capable of generating biosignal(s) indicative of the user's pain level.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A controller (3A) for controlling pumping operation of a milk expression device (3), wherein the controller comprises an input (34) for receiving an input signal indicative of a user's milk ejection reflex, and wherein the controller is configured to generate, in response to the input signal being received, an output for controlling pumping operation of the milk expression device to implement an expression pressure profile comprising a non-zero baseline under-pressure.

2. The controller (3A) according to claim 1, configured to generate the output in response to a milk delivery signal relevant to milk delivery from the user's breast.

3. The controller (3A) according to claim 2, configured to generate a milk delivery-based control signal for setting a level of the non-zero baseline under-pressure based on the milk delivery signal.

4. The controller (3A) according to claim 2 or claim 3, configured to generate the output in response to the milk delivery signal being indicative of a milk delivery parameter reaching or passing a predetermined threshold for at least a predetermined time period.

5. The controller (3A) according to any one of claims 1 to 4, configured to generate, in response to the input signal being received, an output signal for causing the non-zero baseline under-pressure to deepen compared to an initial baseline under-pressure provided prior to the input signal being received.

6. The controller (3A) according to any one of claims 1 to 5, configured to generate, in response to the input signal being received, output signal data for causing the non-zero baseline under-pressure to be maintained during a main period during which the expression pressure profile is implemented, and then to diminish during an end period that follows the main period.

7. The controller (3A) according to any one of claims 1 to 6, configured to generate the output in response to the input signal being indicative of the milk ejection reflex being maintained for at least a predetermined duration; optionally wherein the controller is configured to generate, during the predetermined duration, a control output for controlling pumping operation of the milk expression device to implement an adaptation pressure profile in which one or more pressure-related parameters are progressively adjusted towards the expression pressure profile comprising the non-zero baseline under-pressure.

8. The controller (3A) according to any one of claims 1 to 7, configured to generate the output in response to a milk duct dimension signal relevant to a milk duct dimension.

9. The controller (3A) according to claim 8, configured to generate the output in response to the milk duct dimension signal being indicative of a milk duct dimension parameter reaching or passing a given threshold; and/or wherein the controller is configured to generate a duct dimension-based control signal to set a magnitude of the non-zero baseline under-pressure based on the milk duct dimension signal.

10. The controller (3A) according to any one of claims 1 to 9, configured to generate, in response to the input signal, a further output for controlling one or more mechanical vibration units (36) for vibrating at least part of the user's breast.

11. The controller (3A) according to any one of claims 1 to 10, comprising a further input (38) for receiving a further input signal indicative of a maximum milk flow and/or a degree of discomfort experienced by the user, wherein the controller is configured to generate a control signal to set an upper level of the under-pressure in the expression pressure profile based on the further input signal.

12. A milk expression system (1) comprising:
a milk expression device (3);
the controller (3A) of any one of claims 1 to 11 for controlling pumping operation of the milk expression device; and
a sensor system (30) for generating the input signal indicative of the milk ejection reflex.

13. The milk expression system (1) according to claim 12, wherein the sensor system (30) comprises one or more of
a milk delivery sensor for sensing milk delivery;
a milk duct sensor for sensing a milk duct dimension; and
a skin sensor for sensing one or more skin characteristics relevant to milk ejection reflex.

14. A method (100) for controlling pumping operation of a milk expression device (3), the method comprising:
receiving (102) an input signal indicative of a milk ejection reflex; and
generating (104), in response to the input signal being received, an output for controlling pumping operation of the milk expression device to implement an expression pressure profile comprising a non-zero baseline under-pressure.

15. A computer program comprising computer program code configured to cause a controller, when said code is executed on said controller, to execute the method (100) according to claim 14.
